# EUROPEAN PATENT SPECIFICATION

(11) **EP 1 305 228 B1**
(45) Date of publication and mention of the grant of the patent: **14.04.2004**
(21) Application number: 01959782.2
(22) Date of filing: 09.07.2001
(51) Int. Cl.: B65D 35/36

(54) **APPLICATOR**
APPLIKATOR
APPLICATEUR

(30) Priority: 28.07.2000 US 221641 P
(43) Date of publication of application: 02.05.2003
(73) Proprietor: Pfizer Products Inc., Groton, CT 06340-5146 (US)
(72) Inventor: KNIERIEMEN, Paul, New York, NY 10033 (US)
(74) Representative: Ebner von Eschenbach, Jennifer
(86) International application number: PCT/US2001/041306
(87) International publication number: WO 2002/010029

(56) References cited:
- EP-A- 0 315 513
- DE-U- 7 809 143
- FR-A- 2 581 569
- US-A- 2 118 051
- US-A- 3 121 906
- PATENT ABSTRACTS OF JAPAN vol. 1999, no. 13, 30 November 1999 (1999-11-30) & JP 11 221116 A (SHISEIDO CO LTD), 17 August 1999 (1999-08-17)

## Description

### Background of the Invention

The present invention relates to an applicator according to the preamble of claim 1.

Many consumer products are packaged in small hand held containers with removable closure caps to retain the freshness of the product between uses thereof. However, these container and closure cap assemblies are somewhat complicated in construction and in many instances they simply function to dispense the material within the container.

### Summary of the Invention

The document US 3 121 906 A discloses an applicator according to the preamble of claim 1

Accordingly, it is an object of the present invention to provide an applicator which is simple in construction and simple to use.

Another object of the present invention is an applicator which functions to both dispense and apply the container contents where desired.

In accordance with the present invention, an applicator comprises a flexible container having an upper end portion with at least one dispensing orifice therein. An applicator head of foam material having a plurality of protrusions thereon is secured to the outside surface of the upper end portion of the flexible container whereby substances within the container flow through the foam material of the applicator head when dispensed through the orifice. A closure cap is removably snap fitted over the upper end portion of the container. Releasable locking structure is located on the inside surface of the closure cap and the outside surface of the upper end portion of the flexible container so that the closure cap is releasably secured to the flexible container.

The releasable locking structure preferably includes a plurality of spaced apart outwardly directed lugs on the upper end portion of the flexible container and a plurality of inwardly directed lugs on the inside surface of the closure cap that snap fit with the lugs on the flexible container. The closure cap preferably includes an oval shaped side wall. The upper end portion of the flexible container has a complimentary oval shape.

In a preferred embodiment of the present invention, the applicator head of foam material includes a plurality of raised protrusions equally spaced apart and arranged along an oval path near the outer edge of the applicator head with several additional protrusions positioned within the oval path.

### Brief Description of the Drawings

Novel features and advantages of the present invention in addition to those noted above will become apparent to persons of ordinary skill in the art from a reading of the following detailed description in conjunction with the accompanying drawings wherein similar reference characters refer to similar parts and in which:
Figure 1 is front elevational view of an applicator, according to the present invention, the rear elevational view being identical;
Figure 2 is a right side elevational view of the applicator shown in Figure 1, the left side elevational view being identical;
Figure 3 is a top plan view of the applicator shown in Figures 1 and 2,
Figure 4 is a bottom plan view of the applicator shown in Figures 1-3;
Figure 5 is a front elevational view of the applicator shown in Figures 1-4 with the closure cap removed, the rear elevational view being identical;
Figure 6 is a top plan view of the applicator with the closure cap removed;
Figure 7 is a right side elevational view of the applicator with the closure cap removed, the left side elevational view being identical;
Figure 8 is a bottom plan view of the closure cap shown in Figures 1-4;
Figure 9 is a partial front elevational view similar to Figure 1 but with portions broken away to show interior details; and
Figure 10 is a fragmental enlarged cross-sectional view showing details of the foam head of the applicator.

### Detailed Description of the Invention

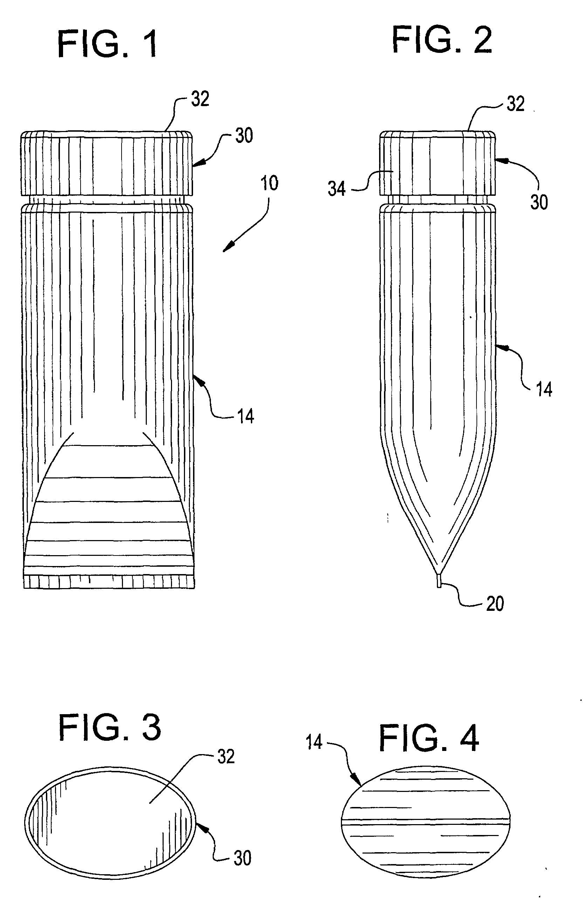

Referring in more particularity to the drawings, Figures 1-8 illustrate the exterior features of an applicator 10 for dispensing and applying materials or substances 12, such as ointments, creams and the like. Applicator 10 may be manufactured from any suitable thermoplastic material compatible with the substance packaged. Such thermoplastic materials include polyethylene and polypropylene, for example. Extrusion and welding techniques, well known in the art, may be used to produce the finished applicator product.

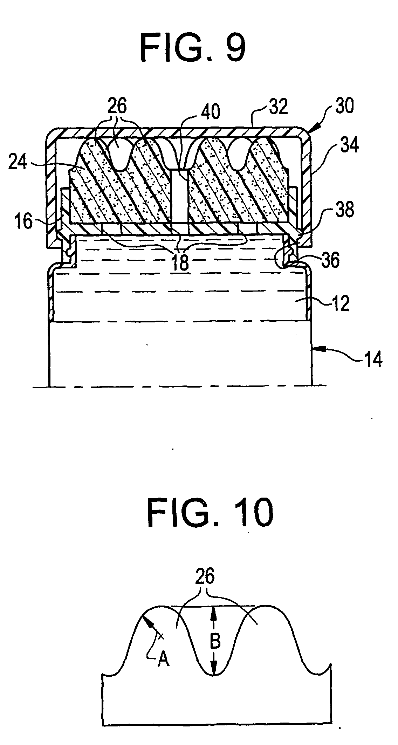

Applicator 10 comprises a flexible container 14 having an upper end portion 16. As shown best in Figure 9, dispensing orifice 18 is provided in the upper end portion 16 of the container. Alternatively, the dispensing orifice may be offset and/or more than one orifice may be used. The lower end of container 14 is closed by a straight seal 20.

The upper end portion 16 of applicator 10 includes an upwardly extending side wall 22, and an applicator head 24 foam material and bearing a plurality of protrusions is secured within the upwardly extending side wall 22, as shown best in Figure 9. The foam material consists of closed cell foam, and the substance 12 flows through a passageway 40 in the foam to the exterior surface of the applicator head when the flexible container 14 is squeezed thereby dispensing the substance through the orifice 18 into the foam material. Alternatively, the material of the applicator head may be selected so that the applicator head is impregnated with substance 12 when the container is squeezed.

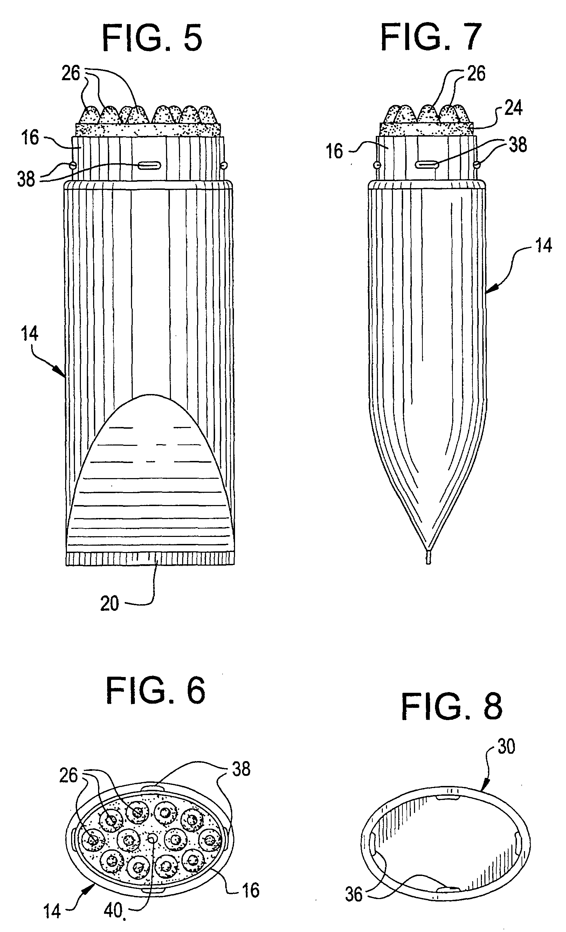

The upper surface of the applicator head includes a plurality of spaced apart bumps or protrusions 26, each having a height B of approximately 3,2.10⁻³ meters to 6,4.10⁻³ meters (1/8 to 1/4 inches) and a circular base dimension having a radius A of 3,2.10⁻³ meters to 6,4.10⁻³ meters (1/8 to 1/4 inches). As shown best in Figure 6, the dimples 26 are equally spaced apart and arranged along an oval path near the outer edge of the applicator head with several additional protrusions positioned within the oval path. Although twelve protrusions are shown, if the protrusions are larger, a lesser number may be sufficient. Also, if the protrusions are smaller, more protrusions may be desirable.

Applicator 10 also includes a flexible closure cap 30 having a generally flat top 32 and a downwardly extending surrounding side wall 34. Closure cap 30 snap fits over the upper end portion 16 of the flexible container to releasably secure the closure cap to the container. Releasable locking structure providing the snap fit is located on the inside of the surrounding wall 34 and on the outside of the upper end portion 16. The locking structure on the inside of a surrounding wall 34 includes four equally spaced apart inwardly extending lugs 36, and the locking structure on the outside surface of the upper end portion 16 includes four equally spaced apart outwardly extending lugs 38 in vertical alignment with the lugs 36 on the closure cap.

In the preferred embodiment of the present invention, the surrounding wall 34 of the closure cap has an oval shape and the upper end portion of the flexible container has a complimentary oval shape. Accordingly, whenever the closure cap is fitted over the upper end portion of the flexible container, the lugs 36 and 38 are always in vertical alignment with one another to produce the desired interference with one another which produces the snap fit.

In use, the closure cap is first removed from the upper end portion of the flexible container to expose the applicator head 24 of foam material. Container 14 is then gently squeezed to dispense substance 12 through orifice 18 into the foam material to the exterior surface thereof. The applicator head 24 is then rubbed over the desired area to stimulate that area and simultaneously apply substance 12. Upon completion of the application process, the closure cap is returned and snap fitted over the upper end portion of the container to preserve the freshness of the packaged substance.

## Claims

1. An applicator (10) comprising a flexible container (14) and a closure cap (30), the flexible container (14) having an upper end portion (16) with at least one dispensing orifice (18) therein, an applicator head (24) of foam material being secured to an outside surface of the upper end portion (16) whereby substances (12) within the flexible container (14) flow through the foam material of the said applicator head (24) when dispensed through the orifice (18), and releasable locking structure on an inside surface of the closure cap (30) and an outside surface of the upper end portion (16) of the flexible container (14) whereby the closure cap (30) is releasably secured to the flexible container (14) **characterised in that** the applicator head (24) of foam material has a plurality of protrusions (26) thereon.

2. An applicator (10) as in claim 1, wherein the releasable locking structure includes a plurality of spaced apart outwardly directed lugs (38) on the upper end portion (16) of the flexible container (14) and a plurality of inwardly directed annular lugs (36) on the inside surface of the closure cap (30) that snap fit with the lugs on the flexible container (14).

3. An applicator (10) as in claim 1, wherein the closure cap (30) includes an oval shaped side wall, and wherein the upper end portion (16) of the flexible container (14) has a complementary oval shape.

4. An applicator (10) as in claim 1, wherein the dimpled applicator head (24) of foam material includes a plurality of protrusions (26) equally spaced apart and arranged along an oval path near an outer edge of the applicator head (24) with several additional protrusions positioned within the oval path.

## Patentansprüche

1. Applikator (10), umfassend einen flexiblen Behälter (14) und eine Verschlusskappe (30), wobei der flexible Behälter (14) einen oberen Endabschnitt (16) aufweist mit mindestens einer Düse (18) darin zum Dispensieren, einen Applikatorkopf (24) aus Schaumstoff, der an der Außenseite des oberen Endabschnittes (16) befestigt ist, wodurch die Substanzen (12) im Inneren des flexiblen Behälters (!4) durch den Schaumstoff des Applikatorkopfes (24) fließen, wenn sie durch die Düse (18) dispensiert werden; sowie eine lösbare Einspannkonstruktion an der Innenseite der Verschlusskappe (30) und einer Außenseite des oberen Endabschnittes (16) des flexiblen Behälters (!4), wodurch die Verschlusskappe (30) lösbar an dem flexiblen Behälter (14) befestigt ist, **dadurch gekennzeichnet, dass** der Applikatorkopf (24) aus Schaumstoff eine Mehrzahl von Vorsprüngen (26) darauf hat.

2. Applikator (10) nach Anspruch 1, worin die lösbare Einspannkonstruktion eine Mehrzahl beabstandeter, nach außen gerichteter Ansätze (38) auf dem oberen Endabschnitt (16) des flexiblen Behälters (14) aufweist und eine Mehrzahl nach innen gerichteter ringförmiger Ansätze (36) auf der Innenseite der Verschlusskappe (30), die mit den Ansätzen an dem flexiblen Behälter (14) eine Schnappverbindung bilden

3. Applikator (10) nach Anspruch 1, worin die Verschlusskappe (30) eine oval geformte Seitenwand einschließt und worin der obere Endabschnitt (16) des flexiblen Behälters (!4) eine komplementäre ovale Form hat.

4. Applikator (10) nach Anspruch 1, worin der leicht vertiefte Applikatorkopf (24) aus Schaumstoff eine Mehrzahl von Vorsprünge (26) einschließt, die gleichmäßig beabstandet sind und nahe eines Außenrandes des Applikatorkopfes (24) auf einer ovalen Linie mit mehreren zusätzlichen, im Inneren der ovalen Linie positionierten Vorsprüngen angeordnet sind.

## Revendications

1. Applicateur (10) comprenant un conteneur souple (14) et un capuchon de fermeture (30), le conteneur souple (14) possédant une partie terminale supérieure (16) ayant au moins un orifice de distribution (18), une tête (24) d'applicateur faite de matière en mousse qui est fixée à la surface externe de la partie terminale supérieure (16), si bien que des substances (12) se trouvant à l'intérieur du conteneur souple (14) s'écoulent à travers la matière en mousse de ladite tête d'applicateur (24) lors de la distribution faite via l'orifice (18), et une structure de blocage libérable sur la surface interne du capuchon de fermeture (30) et la surface externe de la partie terminale supérieure (16) du conteneur souple (14) si bien que le capuchon de fermeture (30) est fixé de manière libérable au conteneur souple (14), **caractérisé en ce que** la tête d'applicateur (24) de matière en mousse possède une pluralité de parties saillantes (26).

2. Applicateur (10) selon la revendication 1, où la structure de blocage libérable comporte une pluralité de pattes (38) dirigées vers l'extérieur et mutuellement séparées sur la partie terminale supérieure (16) du conteneur souple (14) et une pluralité de pattes annulaires (36) dirigées vers l'intérieur sur la surface interne du capuchon de fermeture (30), qui s'ajustent par enclenchement brusque avec les pattes se trouvant sur le conteneur souple (14).

3. Applicateur (10) selon la revendication 1, où le capuchon de fermeture (30) comporte une paroi latérale de forme ovale et où la partie terminale supérieure (16) du conteneur souple (14) possède une forme ovale complémentaire.

4. Applicateur (10) selon la revendication 1, où la tête d'applicateur bossuée (24) de matière en mousse comporte une pluralité de parties saillantes (26) qui sont uniformément séparées et sont disposées le long d'un trajet oval proche d'un bord externe de la tête d'applicateur (24), plusieurs parties saillantes supplémentaires étant placées à l'intérieur du trajet oval.
